# EUROPEAN PATENT APPLICATION

(11) **EP 4 763 167 A1**
(43) Date of publication of application: **24.06.2026**
(21) Application number: 25220361.7
(22) Date of filing: 03.12.2025
(51) Int. Cl.: A61F 13/15, A61F 13/533

(54) **A METHOD FOR THE PRODUCTION OF AN ABSORBENT STRUCTURE FOR SANITARY ARTICLES AND RELATED ABSORBENT STRUCTURE**

(30) Priority: 20.12.2024 IT 202400029400
(71) Applicant: Fameccanica.Data S.p.A., 66020 San Giovanni Teatino (CH) (IT)
(72) Inventor: D'APONTE, Francesco, I-66020 San Giovanni Teatino (Chieti) (IT); SABLONE, Gabriele, I-66020 San Giovanni Teatino (Chieti) (IT); PASSARELLA, Fabio, I-66020 San Giovanni Teatino (Chieti) (IT)
(74) Representative: Buzzi, Notaro & Antonielli d'Oulx S.p.A.

(57) **Abstract**

Absorbent structure comprising an absorbent core (10) having at least two non-through channels (18) with an embossed bottom.

## Description

### Field of the invention

The present invention relates to a method for the production of an absorbent structure for sanitary articles.

The invention has been developed particularly with a view to its application to the production of absorbent sanitary articles such as, for example, diapers and training pants for children, absorbent articles for incontinent adults, feminine hygiene pads, and similar sanitary articles intended to absorb body liquids.

According to another aspect the invention relates to an absorbent structure for sanitary articles.

### Background of the invention

Absorbent sanitary articles usually have a layered structure comprising a liquid-impermeable outer web or backsheet, a liquid-permeable inner web or topsheet intended to be placed in contact with the user's skin, and an absorbent structure that has the function of capturing and storing body liquids.

The absorbent structure often comprises cellulose fluff mixed with granular superabsorbent materials. Such superabsorbent materials are known under different names such as, for example, SAP (*Super Absorbent Polymer*) or AGM (*Absorbent Gelling Material*)*.* In most cases, these are hydrogel-forming materials capable of absorbing and capturing liquids.

In absorbent sanitary articles, the absorbent cores are often designed with through channels, i.e., elongated openings that pass through the entire thickness of the absorbent core, extending from a first surface to a second surface of the absorbent core. These channels are commonly used to reduce the liquid acquisition time, promoting rapid transport of fluids from the surface in contact with the user's body towards the inner layers of the product. The presence of through channels in fact allows the creation of preferential paths for the fluid, improving the efficiency in the acquisition and distribution of the liquid within the absorbent core.

However, such solutions can present disadvantages such as a reduction in the structural strength of the absorbent core. In fact, the through channels create areas devoid of material inside the absorbent core, weakening its overall structure. This can cause deformation of the absorbent core during use, loss of the ability to keep the liquid trapped, with a greater risk of leakage, and non-uniform distribution of the liquid, with the risk that the liquid accumulates in specific areas, compromising user comfort.

From the point of view of the production process, the precise creation of areas devoid of absorbent material requires the accurate removal of the absorbent material in the designated areas. This can be complex and require specific equipment, increasing the costs and complexity of the production process. Any inaccuracy in the removal of the material can influence the functionality of the channels, compromising their effectiveness in fluid management.

### Object and summary of the invention

The object of the present invention is to provide a method for the production of an absorbent structure that overcomes the problems of the prior art.

According to the present invention, this object is achieved by a method having the features forming the subject of claim 1.

According to another aspect, the invention relates to an absorbent structure having the features of claim 6.

The claims form an integral part of the technical teaching provided in relation to the invention.

### Brief description of the drawings

The invention will now be described in detail with reference to the accompanying drawings, given by way of non-limiting example only, in which:
- figure 1 is a schematic plan view of a first embodiment of an absorbent structure according to the present invention,
- figures 2 and 3 are schematic sections along lines II-II and III-III of figure 1,
- figure 4 is a schematic view illustrating on a larger scale the detail indicated by arrow IV in figure 1,
- figure 5 is a schematic plan view of a second embodiment of an absorbent structure according to the present invention,
- figures 6 and 7 are schematic sections along lines VI-VI and VII-VII of figure 5,
- figure 8 is a schematic front view of a machine for the production of absorbent structures according to figures 1-3, and
- figure 9 is a schematic front view of a machine for the production of absorbent structures according to figures 5-7.

It will be appreciated that the various figures may not be drawn to the same scale. It will also be appreciated that some elements or components may not be illustrated in order to make others more visible and to simplify the understanding of the figures.

### Detailed description

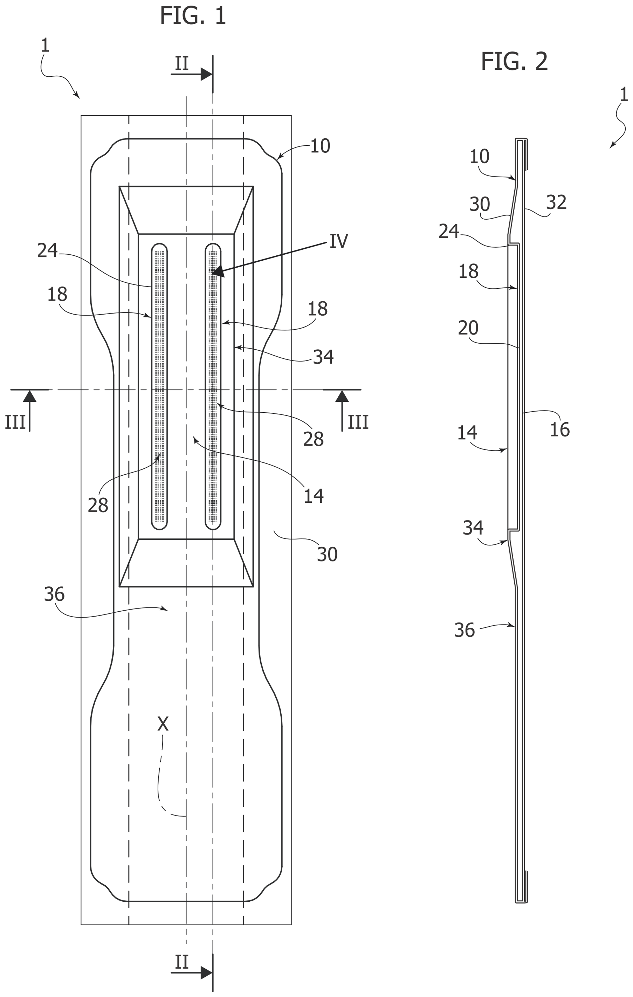

With reference to figures 1-4, numeral 1 indicates an absorbent structure for an absorbent sanitary article according to a first embodiment of the present invention.

The absorbent structure 1 comprises an absorbent core 10 consisting of a mixture of cellulose fibers and granular superabsorbent polymer. The absorbent core 10 has a thin elongated shape in a longitudinal direction X and has a first surface 14 and a second surface 16 opposite to each other.

The absorbent core 10 comprises at least two non-through channels 18 having an elongated shape.

The non-through channels 18 can have any shape and relative arrangement. In the form illustrated by way of example in the figures, the absorbent core 10 has at least two non-through channels 18 arranged parallel to each other. The non-through channels 18 can extend parallel to the longitudinal direction X.

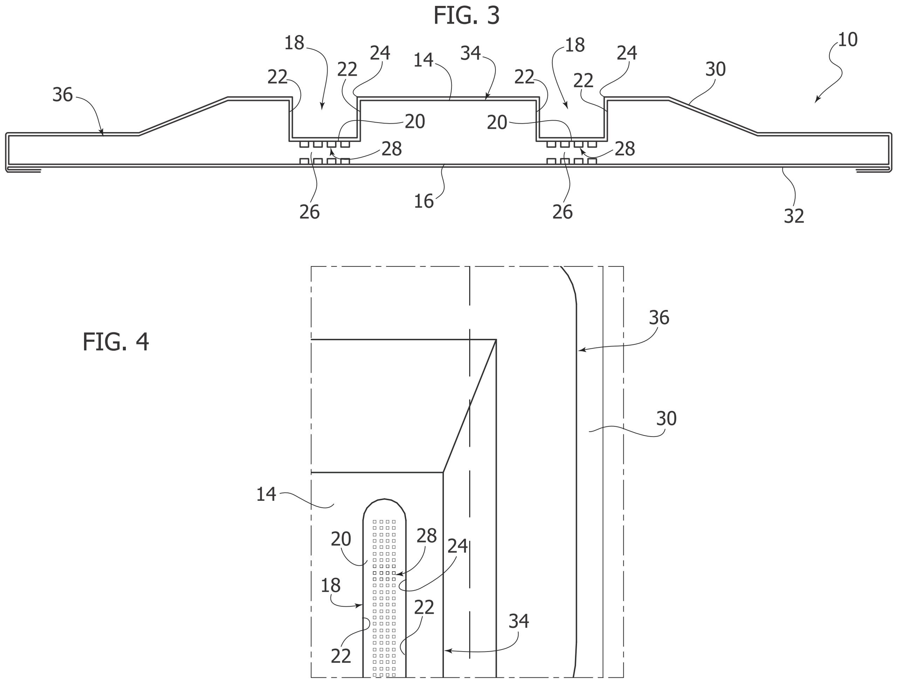

With reference to figure 3, each channel 18 has a bottom surface 20, two side surfaces 22 and an edge 24 open on the first surface 14.

Between the bottom surface 20 of each channel 18 and the second surface 16 of the absorbent core 10, a layer of absorbent material 26 is arranged. The layer of absorbent material 26 is compressed by an embossing pattern 28, illustrated in greater detail in figure 4.

The embossing pattern 28 can be formed by discrete elements separated from each other. The embossing pattern 28 is formed by a process in which the layer of absorbent material 26 is subjected to pressure through a specific pattern applied by embossing rolls. The embossing process exerts pressure in defined areas of the layer of absorbent material 26, creating compressed areas with a higher density compared to the non-compressed areas. The embossing rolls have surfaces with raised patterns (e.g., lines, grids, or geometric shapes) . When these patterns are impressed onto the fiber layer, compressed zones are formed that follow the pattern of the raised surfaces.

The fiber compression achieved by the embossing process improves the cohesion of the fibers, giving greater mechanical strength to the absorbent core. In cases where multiple layers of material are present (such as nonwoven fabric layers), embossing can be used to fix these layers together. Compression by embossing increases the local density of the material and improves the overall structural integrity.

In a possible embodiment, the absorbent core 10 can be enclosed between a first nonwoven fabric web 30 and a second nonwoven fabric web 32, which extend in contact with the first surface 14 and the second surface 16 of the absorbent core 10, respectively.

The first nonwoven fabric web 30 and the second nonwoven fabric web 32 can be fixed to the first surface 14 and the second surface 16 of the absorbent core 10, respectively, by glue.

With reference to figures 2 and 3, the first nonwoven fabric web 30 can also cover the side surfaces 22 and the bottom surface 20 of each channel 18. The first nonwoven fabric web 30 and the second nonwoven fabric web 32 can be fixed to the layer of absorbent material 26 that extends between the bottom surface 20 of each channel 18 and the second surface 16 of the absorbent core 10 by the embossing pattern 28.

The absorbent core 10 can include a central zone 34 in which at least two channels 18 are formed. The central zone 34 can have a basis weight between 300 and 600 gsm, while the layer of absorbent material 26 compressed by the embossing pattern 28 can have a basis weight between 80 and 180 gsm.

In the embodiment illustrated in figures 1-4 the absorbent core 10 can comprise an outer zone 36 that surrounds the central zone 34. The outer zone 36 can have a basis weight lower than that of the central zone 34, for example comprised between 180 - 250 gsm. These basis weight values are indicated by way of example only and must not be interpreted as a limitation of the scope of the present invention.

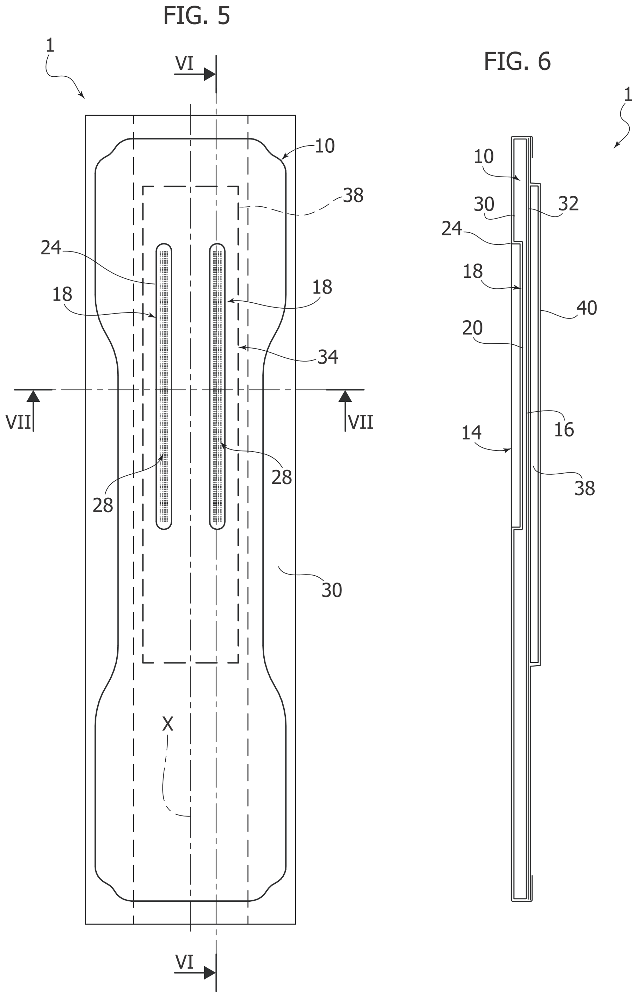

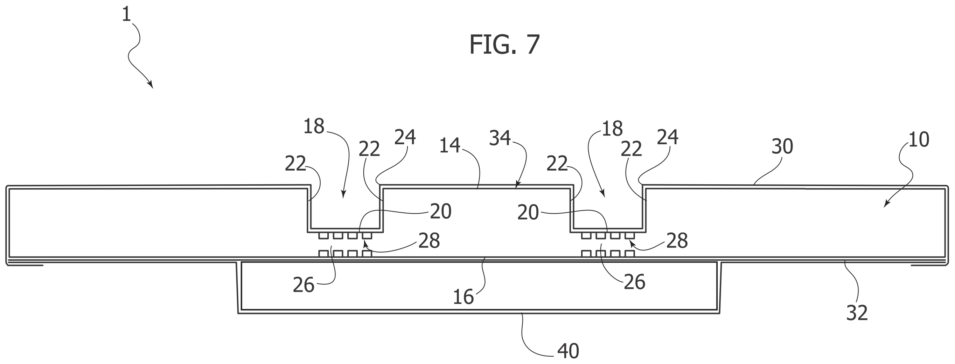

Figures 5-7 illustrate a second embodiment of an absorbent structure 1 according to the present invention. The elements corresponding to those previously described are indicated by the same reference numerals.

In this second embodiment, the absorbent structure 1 comprises an absorbent core 10 having a basis weight comprised, for example, between 180 - 350 gsm. The absorbent structure 1 comprises an auxiliary absorbent pad 38 facing the second surface 16 of the absorbent core 10. The auxiliary absorbent pad 38 can be enclosed between the second nonwoven fabric web 32 and a third nonwoven fabric web 40, which can be fixed to the auxiliary absorbent pad 38 and to the second nonwoven fabric web 32 by glue. The auxiliary absorbent pad 38 can contribute to increasing the overall absorbent capacity and to improving liquid distribution.

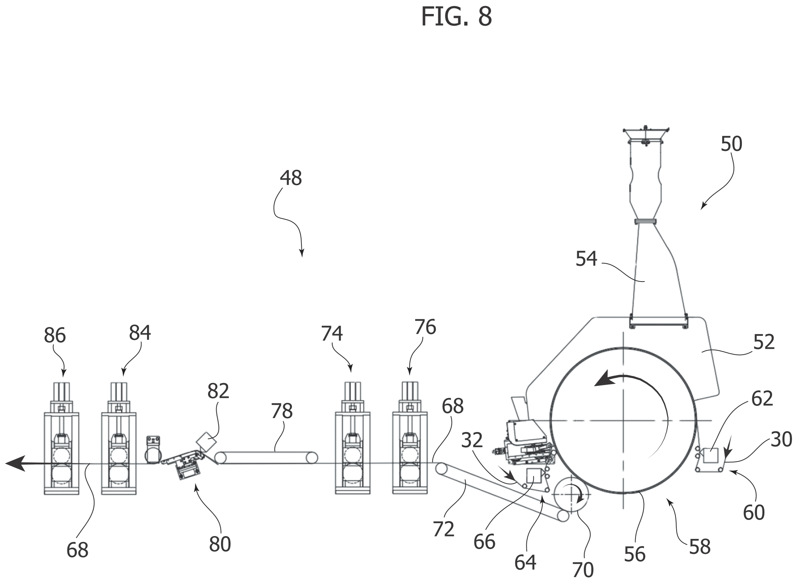

Figure 8 schematically illustrates a portion of a machine 48 for the production of an absorbent structure 1 according to the embodiment of figures 1-4.

The machine portion 48 comprises a forming apparatus 50 including a deposition chamber 52, which distributes a mixture of cellulose fluff and granular superabsorbent polymer coming from a feed channel 54 onto a forming surface 56 provided with discrete forming seats each having a shape complementary to that of the absorbent core 10. The forming surface 56 can be the outer surface of a forming wheel 58, which can be rotatable about a horizontal axis.

The machine 48 can include a first feeder 60 for feeding a first continuous nonwoven fabric web 30 onto the forming surface 56 upstream of the forming chamber 52. A first glue applicator 62 can be provided for applying glue onto the first continuous nonwoven fabric web 30. The machine 48 can include a second feeder 64 that feeds a second continuous nonwoven fabric web 32 onto the forming surface 56 downstream of the forming chamber 52. A second glue applicator 66 can be provided for applying glue onto the second continuous nonwoven fabric web 32.

The forming apparatus 50 forms a continuous composite web 68 comprising an array of absorbent cores 10 enclosed between a first continuous nonwoven fabric web 30 and a second continuous nonwoven fabric web 32.

A stripping wheel 70 detaches the continuous composite web 68 from the forming surface 56 and sends it onto a first conveyor 72.

The first conveyor 72 feeds the continuous composite web 68 to an embossing unit 74 that performs the embossing of the bottom walls of the channels 18 of the array of absorbent cores 10. Upstream of the embossing unit 74 a first pressing unit 76 can be arranged that presses the edges of the array of absorbent cores 10.

Downstream of the embossing unit 74 a second conveyor 78 can be arranged that can feed the continuous composite web 68 to an edge folding unit 80 that can perform the folding of the lateral edges of the first and second continuous nonwoven fabric webs 30, 32. Upstream of the edge folding unit 80 a third glue applicator 82 can be arranged that can apply glue on the edges of the lateral edges of the first and second continuous nonwoven fabric webs 30, 32.

Downstream of the edge folding unit 80 a second and a third pressing unit 84, 86 can be arranged that can press the zones with higher basis weight 34 and the zones with lower basis weight 36 of the array of absorbent cores 10, respectively.

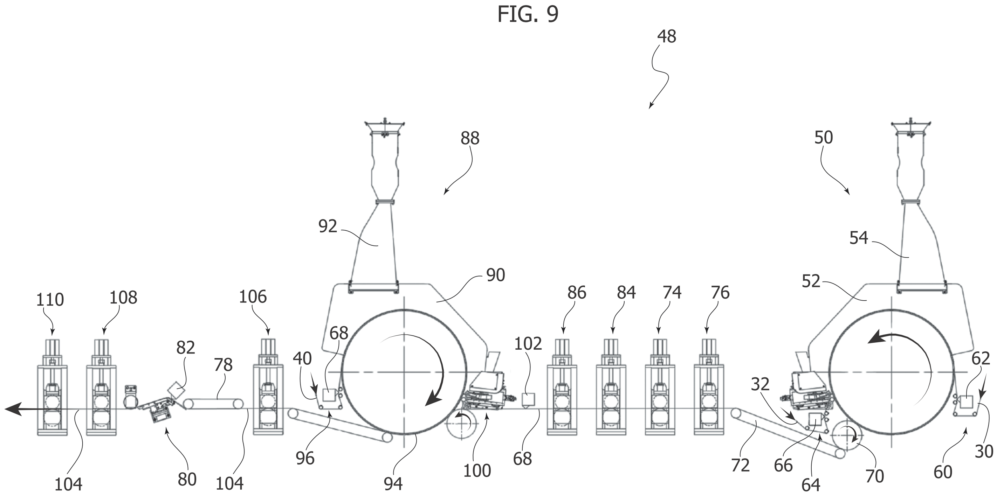

Figure 9 schematically illustrates a portion of a machine 48 for the production of an absorbent structure 1 according to the embodiment of figures 5-7. The elements corresponding to those previously described are indicated with the same reference numerals.

In this embodiment the machine portion 48 comprises a second forming apparatus 88 including a deposition chamber 90, which distributes a mixture of cellulose fluff and granular superabsorbent polymer coming from a feed channel 92 onto a forming surface 94 provided with discrete forming seats each having a shape complementary to that of the auxiliary absorbent pad 38.

The machine portion 48 can include a third feeder 96 for feeding a third continuous nonwoven fabric web 40 onto the forming surface 56 upstream of the forming chamber 90. A fourth glue applicator 98 can be provided for applying glue onto the third continuous nonwoven fabric web 40. The machine 48 can include a fourth feeder 100 that feeds the continuous composite web 68 onto the forming surface 94 downstream of the forming chamber 52. A fifth glue applicator 102 can be provided for applying glue onto the continuous composite web 68 upstream of the fourth feeder 100.

The second forming apparatus 88 forms an array of auxiliary absorbent pads 38 that are applied to respective absorbent cores 10. The auxiliary absorbent pads 38 are enclosed between the second continuous nonwoven fabric web 32 and the third continuous nonwoven fabric web 40 forming a second continuous composite web 104.

Downstream of the second forming apparatus 88 further pressing units 106, 108, 110 can be arranged to complete the fixing between the auxiliary absorbent pads 38 and the respective absorbent cores 10, and to press the zones with higher basis weight and the zones with lower basis weight of the second continuous composite web 104.

In operation the previously described machine portion 48 implements a method for the production of an absorbent structure, comprising:
- forming an absorbent core 10 including a mixture of cellulose fibers and granular superabsorbent polymer, wherein the absorbent core 10 has at least two non-through channels 18 with an elongated shape each having a bottom surface 20, two side surfaces 22 and an edge 24 open on the first surface 14 of the absorbent core 10, wherein the absorbent core 10 has a layer of absorbent material 26 arranged between the bottom surface 20 of each channel 18 and the second surface 16 of the absorbent core; and
- compressing said layer of absorbent material 26 arranged between the bottom surface 20 of each channel 18 and the second surface 16 of the absorbent core 10 by an embossing pattern 28.

The embossing pattern 28 may be formed by discrete elements separated from each other.

The method may comprise forming at least two non-through channels 18 parallel to each other in the absorbent core 10.

The method may comprise enclosing the absorbent core 10 between a first nonwoven fabric web 30 and a second nonwoven fabric web 32 that extend in contact with the first surface 14 and the second surface 16 of the absorbent core 10, respectively.

The method may comprise covering with the first nonwoven fabric web 30 the side surfaces 22 and the bottom surface 20 of each channel 18, and fixing by the embossing pattern 28 the first and the second nonwoven fabric webs 30, 32 to the layer of absorbent material 26 arranged between the bottom surface 20 of the channel 18 and the second surface 16 of the absorbent core 10.

The method may provide that at least two channels 18 are formed in a central zone 34 of the absorbent core 10 having a basis weight between 300 and 600 gsm and that the embossing pattern 28 compresses said layer of absorbent material 26 to a basis weight between 80 and 180 gsm.

The method may comprise the formation of an outer zone 36 that surrounds the central zone 34 having a basis weight between 180-250 gsm.

The method may further comprise the step of disposing an auxiliary absorbent pad 38 facing the second surface 16 of the absorbent core 10 and enclosing it between the second nonwoven fabric web 32 and a third nonwoven fabric web 40.

An aspect of the present invention is an absorbent structure obtained by a method comprising:
- forming an absorbent core 10 including a mixture of cellulose fibers and granular superabsorbent polymer, wherein the absorbent core 10 has at least two non-through channels 18 with an elongated shape each having a bottom surface 20, two side surfaces 22 and an edge 24 open on the first surface 14 of the absorbent core 10, wherein the absorbent core 10 has a layer of absorbent material 26 arranged between the bottom surface 20 of each channel 18 and the second surface 16 of the absorbent core; and
- compressing said layer of absorbent material 26 arranged between the bottom surface 20 of each channel 18 and the second surface 16 of the absorbent core 10 by an embossing pattern 28.

The non-through channels with embossed bottom according to the present invention offer numerous advantages compared to known solutions with through channels, including better structural integrity thanks to the fact that the absorbent material remains continuous on the side opposite the channel. This preserves the structural integrity of the absorbent core, reducing the risk of deformations or breakage during use.

The presence of an embossed bottom layer ensures that the fibers and the superabsorbent polymer remain well distributed and cohesive, avoiding the risk of material dispersion during use or handling.

The non-through channels with embossed bottom facilitate the distribution of fluids along the absorbent core without allowing a direct passage. This helps to spread the liquid uniformly, improving comfort and reducing local liquid accumulation.

The embossing of the channel bottom creates compressed zones that favor the horizontal diffusion of liquids within the absorbent core, improving the efficiency of distribution.

From the point of view of the production process, the realization of non-through channels is simpler compared to through channels, as it does not require the complete removal of the absorbent material. This simplifies the production process, reduces the risk of contamination from material residues and reduces production waste.

Embossing allows selective compression of the layer of absorbent material at the desired points, guaranteeing a precise configuration of the channels without complicating the production process.

Naturally, the principle of the invention remaining the same, the details of construction and the embodiments may be varied with respect to what has been described and illustrated without thereby departing from the scope of the invention as defined by the claims that follow.

### List of reference numerals

1: Absorbent structure
10: Absorbent core
14: First surface of the absorbent core
16: Second surface of the absorbent core
18: Non-through channel
20: Bottom surface of the channel
22: Side surfaces of the channel
24: Open edge of the channel on the first surface
26: Layer of absorbent material
28: Embossing pattern
30: First nonwoven fabric web
32: Second nonwoven fabric web
34: Central zone of the absorbent core
36: Outer zone of the absorbent core
38: Auxiliary absorbent pad
40: Third nonwoven fabric web
48: Machine portion for the production of absorbent structures
50: Forming apparatus
52: Deposition chamber
54: Feed channel
56: Forming surface
58: Forming wheel
60: First feeder
62: First glue applicator
64: Second feeder
66: Second glue applicator
68: Continuous composite web
70: Stripping wheel
72: First conveyor
74: Embossing unit
76: First pressing unit
78: Second conveyor
80: Edge folding unit
82: Third glue applicator
84: Second pressing unit
86: Third pressing unit
88: Second forming apparatus
90: Deposition chamber of the second forming apparatus
92: Feed channel of the second forming apparatus
94: Forming surface of the second apparatus
96: Third feeder
98: Fourth glue applicator
100: Fourth feeder
102: Fifth glue applicator
104: Second continuous composite web
106: Further pressing unit
108: Further pressing unit
110: Further pressing unit

## Claims

1. A method for the production of an absorbent structure, comprising:
- forming an absorbent core (10) including a mixture of cellulose fibers and granular superabsorbent polymer, wherein the absorbent core (10) has at least two non-through channels (18) with an elongated shape each having a bottom surface (20), two side surfaces (22) and an edge (24) open on the first surface (14) of the absorbent core (10), wherein the absorbent core (10) has a layer of absorbent material (26) arranged between the bottom surface (20) of each channel (18) and the second surface (16) of the absorbent core; and
- compressing said layer of absorbent material (26) arranged between the bottom surface (20) of each channel (18) and the second surface (16) of the absorbent core (10) by an embossing pattern (28).

2. The method of claim 1, wherein said embossing pattern (28) is formed by discrete elements separated from each other.

3. The method of any one of the preceding claims, comprising enclosing the absorbent core (10) between a first nonwoven fabric web (30) and a second nonwoven fabric web (32) that extend in contact with the first surface (14) and the second surface (16) of the absorbent core (10), respectively.

4. The method of claim 3, comprising covering with the first nonwoven fabric web (30) the side surfaces (22) and the bottom surface (20) of each channel (18), and fixing the first and the second nonwoven fabric webs (30, 32) to the layer of absorbent material (26) arranged between the bottom surface (20) of each channel (18) and the second surface (16) of the absorbent core (10) by said embossing pattern (28).

5. The method of any one of the preceding claims, further comprising the step of disposing an auxiliary absorbent pad (38) facing the second surface (16) of the absorbent core (10) and enclosing it between the second nonwoven fabric web (32) and a third nonwoven fabric web (40).

6. An absorbent structure comprising an absorbent core (10) formed from a mixture of cellulose fibers and granular superabsorbent polymer, wherein the absorbent core (10) has a first surface (14) and a second surface (16), wherein the absorbent core (10) has at least two non-through channels (18) with an elongated shape each having a bottom surface (20), two side surfaces (22) and an edge (24) open on the first surface (14), wherein between the bottom surface (20) of each channel (18) and the second surface (16) of the absorbent core (10) a layer of absorbent material (26) compressed by an embossing pattern (28) is arranged.

7. The absorbent structure of claim 6, wherein said embossing pattern (28) is formed by discrete elements separated from each other.

8. The absorbent structure of claim 6 or claim 7, wherein the absorbent core (10) is enclosed between a first nonwoven fabric web (30) and a second nonwoven fabric web (32), which extend in contact with the first surface (14) and the second surface (16) of the absorbent core (10), respectively.

9. The absorbent structure of claim 8, wherein the first nonwoven fabric web (30) covers the side surfaces (22) and the bottom surface (20) of each channel (18) and wherein the first and the second nonwoven fabric webs (30, 32) are fixed to the layer of absorbent material (26) arranged between the bottom surface (20) of each channel (18) and the second surface (16) of the absorbent core (10) by said embossing pattern (28).

10. The absorbent structure of any one of claims 6-9, comprising an auxiliary absorbent pad (38) facing the second surface (16) of the absorbent core (10) and enclosed between the second nonwoven fabric web (32) and a third nonwoven fabric web (40).
